# EUROPEAN PATENT APPLICATION

(11) **EP 2 039 759 A1**
(43) Date of publication of application: **25.03.2009**
(21) Application number: 07425590.2
(22) Date of filing: 24.09.2007
(51) Int. Cl.: C12N 5/06, A61K 35/28, A61P 9/10

(54) **Ex-vivo expansion method under clinical grade conditions of haematopoietic stem cells isolated from bone marow to treat ischaemic diseases such as acute myocardial infartcion**

(71) Applicant: Fagioli, Franca, 10126 Torino (IT)
(72) Inventor: Fagioli, Franca, 10126 Torino (IT)

(57) **Abstract**

This invention concerns an expansion method under clinical grade conditions of haematopoietic bone marrow CD34+ stem cells including the phases of: (i) isolation, for immunomagnetic selection, of haematopoietic stem cells from a sample of autologous bone marrow using a closed commercial system CliniMacs; (ii) exposition of haematopoietic stem cells to such conditions to permit expansion and addressing them in a mono-endothelial sense, preparation of cell therapy product resuspended in 10 ml of physiological solution and intracoronary grafting using haemodynamic and heart surgery techniques.

This invention also concerns the clinical use of haematopoietic stem cells of marrow blood obtained to treat ischaemic pathologies such as acute myocardial infarction.

## Description

### Field of the invention

This invention concerns an isolation and ex-vivo expansion method of autologous haematopoietic stem cells, isolated from bone marrow blood to treat patients affected by ischaemic pathologies such as myocardial infarction.

### Technical background of the invention

In recent years, the theory that the heart is an organ consisting of differentiated myocytes unable to regenerate cells has been challenged. It seems increasingly evident that there might be limited regeneration in particular pathophysiologies via the use of resident and circulating stem cells (Wollert et al. Circ Res 2005; Nadal-Ginard et al. Circ Res 2003). The description of endogenous repair mechanisms in patients with myocardial infarction, advanced coronary disease or cardiac insufficiencies suggest that cardiac regeneration is a possible therapy in such pathological settings.

The pathophysiological assumption of cell therapy is represented by the plasticity of adult stem cells (Wagers et al. Cell 2004). Stem cells are self-renewable, are able to transform themselves into specific cell progenitors and differentiate into specialised cell lines. The plasticity of stem cells implies that specific precursor cells transdifferentiate into cell types which are different from their line in reply to various microennviromental stimuli. In the specific case of the heart, when haematopoietic stem cells are transplanted in the myocardium, in experimental murine models of acute myocardial ischaemia, they transdifferentiate into cardiomyocytes and vascular structures with a consequent improvement of heart function and survival (Orlic et al, Nature 2001). Cell fusion, as a means to explain the effects of stem cell implantation in myocardial pathologies, has been proposed as an alternative to stem cell transdifferentiation (Alvarez-Dolado et al. Nature 2003; Nygren et al. Nat Med 2004). However, the mechanisms of the functional effects of cell transplant can be rather more complex and articulated than those mentioned above. Stem cells can release angiogenetic ligands, have an antiapoptotic protective effect on cardiomyocytes and induce the proliferation of endogenic cardiomyocytes by recruiting resident cardiac stem cells (Kocher et al. Nat Med 2001; Kamihata et al. Circulation 2001; Fichs et al. J Am Coll Cardiol 2001; Kinnaird et al. Circ Res 2004; Schuster et al. Am J Physiol heart circ Physiol 2004).

There is, however, aside from the pathophysiological and biocellular mechanisms, a scientific consensus that cell therapy may improve the perfusion and contractile function of myocardial pathologies.

In a clinical setting, some phase 1 and phase 2 studies have shown the feasibility and potential clinical benefits of cell therapy in acute and chronic coronary syndromes. Results obtained in humans suggest that myocardial regeneration and neoangiogenesis may be obtained as a direct or indirect effect of the intracoronary or intramyocardial (epicardial or endocardial) injection of bone marrow stem cells (Wollert et al. Circ Res 2005).

Cell therapy is therefore seen with increasing interest to treat ischaemic heart disease in general and, in particular, acute myocardial infarction, above all in those cases in which the best mechanical and pharmacological therapies do not lead to the functional repair of a damaged myocardium.

At present, however, the following still needs to be clarified:
- *Which patients would benefit most from such treatment.* Various types of patients with acute and chronic heart diseases might benefit from such treatment. The most relevant clinical data have been developed in patients with acute myocardial infarction with phase 1 and 2 studies mainly aimed at evaluating feasibility and safety (Wollert et al. Circ Res 2005). In such a clinical setting, it is evident that, despite the best medical and surgical therapies available, patients with a highly compromised mycardial function would benefit most from such a therapeutic approach, despite relative experimental clinical evidence still in early stages. Only a few clinical studies have been carried out to date in patients with chronic ischaemic heart disease and chronic heart failure with encouraging, but at times, contrasting results (Wollert et al. Circ Res 2005).
- *What the best cell transplant procedures are.* Possible methods are: intracoronary; intramyocardial, epi- or endocardial, systemic intravenous, coronary transvenous. To date, the most widely used transplant technique to collect and transplant cells, perhaps the best compromise between efficacy and complexity and which has provided the most encouraging results in patients affected by acute myocardial infarction, is that of aspiring autologous bone marrow cells, under local or general anaesthesia, from the iliac crest, the *ex-vivo* isolation and purification of mononuclear cells and the intracoronary reinjection of the cells (Wollert et al. Circ Res 2005). An alternative technique, albeit with more controversial results, is the mobilisation of bone marrow stem cells with cytokines, in particular G-CSF o GM-CSF (Wollert et al. Circ Res 2005). Moreover, the best strategy might not be the use of a single method in all clinical cases. It might be best to use the intracoronary approach with acute myocardial infarction. Other surgical methods of intracardiac, endocardial, transcatheter or epicardial injection by open heart surgery or of mobilisation with cytokines (G-CSF and/or GM-CSF) might play a role in patients with ischaemic cardiopathies or chronic heart failure.
- *What the best cell lines to use for therapy are.* In most clinical studies carried out to date, one pragmatic approach is the use of unfractionated mononuclear bone marrow cells containing low percentages of various populations of precursor cells: haematopoietic stem cells, endothelial precursors, mesenchymal cells. The fact that it is easy to take and manipulate the cells ex vivo without complex instruments or laboratory procedures are considered as possible advantages of the use of unfractionated bone marrow cells. There have been very few studies using purified cell populations, and there are still no studies comparing the regenerative capacity of various purified cell populations. There are various precursor cell populations for therapeutical use. *CD34 cells* are identified by the expression on the plasmatic membrane of the marker which identifies a population of haematopoietic precursor cells which theoretically transdifferentiate into myocytes or vascular cells or stimulate, via the paracrine effect, myo- and angiogenesis. A low percentage of CD34 cells are also positive for the CD133 membrane marker. The *endothelial cell progenitors* were originally described by the expression on the plasmatic membrane of CD133 and CD34 haematopoietic markers and by VEGFR-2 endothelial marker (vascular endothelial growth factor receptor-2 or KDR), and also by their capacity to be incorporated in neovascularization sites and to differentiate into *in* situ endothelial cells (Asahara et al. Am J Physiol Cell Physiol 2004). A further different line of endothelial precursors has also be reported. Some studies of *in vitro* cell expansion and a study carried out in our centre on haematological samples of patients with acute myocardial infarction treated conventionally or who underwent mobilisation therapy with cytokines (Marra et al. Eurointer 2006; Bruno et al. Cytokine 2006) showed there is a population of mononuclear CD14+, CD34-, KDR+ precursor cells, with similar endothelial precursor capacity which can mediate their angiogenetic capacity by releasing paracrine factors (Rehman et al. Circulation 2003; Urbich et al. Circ Res 2004). *CD133 cells* are characterised by the expression of the marker on the plasmatic membrane. This marker is expressed on both the haematopoietic stem cells and on the endothelial precursor cells, both of which can initiate ischaemic tissue vascularization (Rafii et al. Nat Med 2003). CD133 cells can integrate in neovascularization sites and differentiate into mature endothelial cells. As CD133 expression is lost in myelomonocytic cells lines, this marker provides an effective method to distinguish the various CD133 endothelial precursors from the CD14/KDR monocyte derived endothelial precursors. As CD133 cells represent less than 1% of mononuclear bone marrow cells only a limited number of these cells can be obtained for therapeutic uses. *Mesenchymal cells* represent a rare population of CD34 and CD133 negative of cells in bone marrow stroma (and in the other mesenchymal tissues), and are 10 times less numerous than bone marrow stem cells. Mesenchymal cells can differentiate into osteocytes, condrocytes and adipocytes. The differentiation of mesenchymal cells in cardiomyocytes has been observed in specific culture conditions and after injection into the myocardium of healthy animals and infracted animals (Makino et al. J Clin Invest 1999; Toma et al. Circulation 2002; Mangi et al. Nat Med 2003). When injected into infarcted myocardial tissue under experimental conditions, mesenchymal stem cells can improve the regional parietal kinetics and prevent the remodelling of the non-infarcted tissue (Mangi et al. Nat Med 2003; Shake et al. Am Thorac Surg 2002). On the other hand, the possible effects of these cells on myocardial perfusion are unknown. *Skeletal myoblasts* or *satellite cells,* are normally quiescent precursor cells in adult muscle fibre. Myoblasts can be isolated from skeletal muscular biopsies and expanded *in vitro.* Myoblasts can develop the properties of striate muscle cells when they are transplanted in the infarct scar and improve myocardial performance even though they do not develop excitation-conduction properties (Wollert et al. Circ Res 2005). *Resident cardiac stem cells* can be expanded *in vitro* from human myocardial biopsies and when they are injected into experimental infarction models in animals they appear to be capable of forming cardiomyocytes and vascular cells with a consequent overall improvement in systolic functions (Beltrami et al. Cell 2003). Research with these cells is still at an early stage and when the abovementioned results are reproducible the cardiac stem cells would seem to be very promising for heart cell therapy.
- *When the best time to inject is after the* acute *event.* The myocardial substratum and the tissue repair process and the post-infarction myocardial remodelling determine the optimal moment for cell application. After a massive myonecrosis and haemorrhage the reperfused myocardium is rapidly invaded by leucocytes and mast-cells, with a matrix generation containing fibrin with a peak between 24 and 72 hours. After 7-10 days from the myocardial infarction, necrotic myocytes are removed with a parallel reduction of neutrophils and monocytes, granulation starts 3 weeks later with a scar being completely formed after 6 weeks. The number of circulating stem cells shows a peak and progressive decrease between 7 and 14 days after the infarction (Wojakowski et al. Circulation 2004; Massa et al. Blood 2005). This peak coincides with the expression of chemiotaxis and cell adhesion modulating factors. The precocious increase of oxygen free radicals in the infarcted tissue might condition cell survival, while the inflammatory cytokines (TNF- , IL-1 , IL-6, IL-10) with their pleiotropic effect may contribute to survival or the distribution of myocytes. Unrelated to the often contrasting properties of each cytokine, is the balancing of the inflammatory and antiinflammatory effects which finely regulate the remodelling of the cell matrix, vascular growth and the events which are critical for survival and the function of any implanted stem cells. In conclusion, the optimal moment for cell transplant is probably defined by the balancing between facilitating cytokines and potentially damaging proinflammatory cytokines with homing and cell survival. Considering the progress of repair processes after myocardial infarction and the expression of multiple factors, the highest likelihood of an optimal engraftment and survival of possible transplanted stem cells seems to be between days 3 and 10 from the myocardial infarction (Bartunek et al. Nature 2006).

Based on encouraging data from the base study a few clinical studies were carried out, initially aimed at verifying the feasibility and safety of cell therapy in acute myocardial infarction (phase 1), and were then aimed at evaluating efficacy with a control group (phase 2). All clinical studies included patients with acute myocardial infarction treated with primary angioplasty with stent implants, the stem cells were infused via the intracoronary by using balloon catheters with temporary flow interruptions. These studies used both non-selected bone marrow stem cells (MNC: mononuclear cells) and selected cell populations. As far as concern the clinical studies on non-selected bone marrow cells including stem fractions (all the nucleate cells or only the mononuclear cell fraction)(Strauer et al. Circulation 2002; Assmus et al. Circulation 2002; Schachinger et al. J am Coll Cardiol 2004; Wollert et al. Lancet 2004; Fernandez-Aviles et al. Circ Res 2004; Chen et al. Am J Cardiol 2004), the combined experience of over 100 treated patients means the intracoronary injection of these cells is safe over both the short and the medium terms. No haemorrhage complications either during or after bone marrow collection were recorded. The intracoronary infusion of bone marrow mononuclear cells did not lead to any peri- or postprocedural ischaemic myocardial alterations or any systemic inflammatory reactions (no further significant enzymatic or reactive C protein increases were observed), neither were any increases in post-angioplasty restenosis or ventricular arrhythmia levels noted. No signs of intramyocardial calcification or developments of neoplasms were noted. All the studies (Strauer et al. Circulation 2002; Assmus et al. Circulation 2002; Schachinger et al. J am Coll Cardiol 2004; Wollert et al. Lancet 2004; Fernandez-Aviles et al. Circ Res 2004) showed an improvement in local kinesis, 3 wider studies (Assmus et al. Circulation 2002; Schachinger et al. J am Coll Cardiol 2004; Wollert et al. Lancet 2004; Fernandez-Aviles et al. Circ Res 2004) described an increase of the ejection fraction, even though only one of these included a randomised control group (Wollert et al. Lancet 2004), the improvements in the ejection fraction in this study were mainly due to improvements in regional kinesis at the border zone.

A wider randomised study with interesting indications is the REPAIR-AMI trial. Although this clinical study is yet to be published, the preliminary results have been presented at major international conferences. This trial enrolled 204 patients with acute myocardial infarction and primary angioplasty, randomly treated with an intracoronary injection of mononuclear bone marrow cells (in 2.4x10⁶ media), at 4 days from the acute event. There was a notable improvement in the global ejection fraction of the left ventricle with no major adverse events (Chen et al. Am J Cardiol 2004).

Only two randomised clinical studies were carried out on selected cell lines (bone marrow mesenchymal stem cells (or bone marrow CD133 endothelial precursors) (Bartunek et al. Circulation 2005; Stamm et al. J Thorac Cardiovasc Surg 2007) with contrasting non-definitive results. The poor number of the treated population does not allow any definitive conclusions to be drawn.

A number of our group's *in vitro* and *in vivo* studies in xenogenic transplant models have shown how this is possible:
■ Expand neonatal (cord blood-CB) haematopoietic stem cells and adult haematopoietic tissue (Bone Marrow-BM and Mobilized Peripheral Blood-MPB) with an ex-vivo stroma free culture osystem nly adding exogenous cytokines such as FLT3 ligand (FL), c-kit ligand or Stem Cell Factor (SCF), growth factor for megacaryocytes or thrombopoietin (TPO), interleukine-6 (IL6), with a consequent many-folded increase in the number of haematopoietic progenitors (CFC colony forming cells, LTC-IC)(Piacibello et al. Blood 1997; Piacibello et al. Leucemia 1998; Gammaitoni et al. Exp Hemat 2003).
■ Obtain a complete long term engraftment of NOD/SCID mice transplanted with expanded ex-vivo cells with a consequent increase of the frequency of more primitive stem cells identifiable in-vivo (Scid Repoppulating cells-SRC)(Piacibello et al. 1999).
■ Obtain an imposing expansion and increase of the frequency of SRC without a consequent aging of the cells (Gammaitoni et al. Blood 2004).

These data show how the ex-vivo expansion procedure has been validated with several experiments carried out on hundreds of myeloablate xenorecipients such as NOD/SCID mice and suggest the possibility of a new transplant approach.

Having evaluated these premises of cell pathophysiology and biology and on the basis of clinical data from experimental trials, exploiting a commercial clinical grade selection, we have produced an ex-vivo bone marrow stem cell expansion system for use in therapeutical protocols for patients affected by ischaemic pathologies such as myocardial infarction.
- The originality of our research consists in a one week expansion, in a mixture of carrier-free SCF+FL+TPO+IL6 growth factor, of CD34+ bone marrow cells in order to increase their number without losing the stem cell characteristics and, at the same time, direct them to a mono-endothelial stem cell phenotype. In regenerative medicine of myocardial infarction our innovative invention will permit the use of:
- A stem cell population, isolated from bone marrow, purified, controlled and expanded *in vitro* with well defined cell concentrations and types.
- An injection method which has to date been more effective in patients with acute myocardial infarction: an intracoronary transcatheter injection in a temporary arrest of coronary flow.

### Compendium of the invention

The intracoronary implant of stem cells in patients with acute myocardial infarction and depressed left ventricular function may allow a functional repair of the myocardial area which would otherwise be irreversibly lost: a repair of systolic functions allows an improvement in the functional class of the patient and a positive outlook on the short and long term prognosis. Clinical studies carried out to date have used mononuclear cells derived from bone marrow containing varying percentages of stem cells, or only cells purified for the CD133 antigen with very encouraging results in terms of functional repair. A very recent pre-clinical study suggested the selection of CD34 bone marrow, but unmanipulated, cells (Griesel et al. Cytotherapy 2007) without, however, offering any data on the potential function of such cells in repairing myocardial damage after acute infarction. One of the most interesting scientific and innovative aspects of our invention is the use of the cell manipulation technology to be used.

The selection of CD34⁺ haematopoietic stem cells from the collection of bone marrow provides a highly purified cell population and avoids contaminating the other bone marrow cells, for some of which there is a known proinflammatory role which might be damaging for the myocardial infarction victim, and for others their real role in cardiac repair is still to be ascertained. The innovative aspect of our invention centres on the ***expansion*** procedure, after selecting the CD34⁺ cells, to obtain a greater number of stem cells to inject into the site of the cardiac infarction and at the same time, from the data obtained in our laboratory, direct cells in culture to a stem cell phenotype, which on one hand are more immature than those cells isolated at time zero (cells with a CD34⁺CD133⁺ phenotype) and, on the other hand, potentially more towards a mono-endothelial stem cell line, which from recent literature would seem to be more involved in the angiogenetic repair process of the damage caused by acute myocardial infarction (cells with CD14⁺CD34⁻; CD14⁺CD105⁺; CD14⁺CD31⁺; CD14⁺KDR⁺ phenotypes) (Bruno et al. Cytokyne 2006).

According to this invention, these objectives are reached thanks to the solutions specifically mentioned in the claims section below. The claims are an integral part of the technical explanations supplied here in relation to the invention.

### Specific description of the invention

Following is a detailed description of this invention with reference to a form of implementation at the preferred time through not limiting examples.

This solution provides a new method for the manipulation, of CD34+ haematopoietic stem cells from marrow blood, as laid down in the *"*Guide lines for the beginning of phase I/II clinical studies with living human cells for somatic cell therapy" (Italian Health Ministry Report, volume 12, 1997) and in *"*Guide lines for tissue engineering and cell therapy (Italian Health Ministry Report, volume 12, 1999) and "ISTSAN Report" (01/22, 2001). From this ex-vivo culture method a cell phenotype can be obtained which is potentially useful in the repair of ischaemic damage such as that caused by acute myocardial infarction.

More specifically, one form of implementation preferred by this invention concerns an expansion method of CD34+ haematopoietic stem cells including the phases (i) isolation from a bone marrow sample, via immunomagnetic selection, of haematopoietic stem cells expressing the CD34 surface antigen, via a closed commercial system CliniMacs, and (ii) exposing the recuperated CD34+ stem cells to such conditions to allow the expansion of the more primitive haematopoietic precursors and at the same time, without them losing their distinctive stem cell characteristics or the mono-endothelial immunophenotype cability.
The selection of CD34⁺ haemtaopoietiche stem cells collected from bone marrow provides a highly purified cell population and avoids contaminating the other bone marrow cells, for some of which there is a known proinflammatory role, a negative phenomenon for the myocardial infarction, and for others their real role in cardiac repair is still to be ascertained.
The expansion procedure, after selecting the CD34⁺ cells provides a greater number of stem cells to be injected into the site of the cardiac infarction and at the same time, from the data obtained in our laboratory, direct cells in culture to a stem cell phenotype, which on one hand are more immature than those cells isolated at time zero (cells with a CD34⁺CD133⁺ phenotype) and, on the other, potentially more towards a mono-endothelial stem cell line, which from recent literature would seem to be more involved in the repair process of the damage caused after acute myocardial infarction (cells with CD34+CD133+; CD14+CD34-; CD14+CD105+;CD31+CD105+; CD31+KDR+ phenotypes) (Bruno et al. Cytokine 2006).

The type of implementation at present favoured by this invention, concerns, more precisely, a method to expand haemopoietic bone marrow with the following operations:
(a) isolation of stem cells expressing the CD34+ antigen via immunomagnetic selection with the closed CliniMacs system (Miltenyi Biotec);
(b) expansion, in clinical grade conditions, of CD34+ cells obtained, at a concentration 50-100.000 cells/ml, in a stroma and antibiotic free culture system in Iscove culture medium Modified Dulbecco Medium (IMDM Invitrogen)) added by 10% of bovine foetal serum treated for 30 minutes at 56°C (FBS Defined-Hyclone, EMEA approved) and by the following combinations of carrier-free ex-vivo growth factors: c-kit ligand (SCF) [50ng/ml] + Fl-3/Flk2 ligand (FL)[50ng/ml]+Trombopoitin (TPO)[10ng/ml]+Interleukine-6 (IL-6)[10ng/ml] (all R&D systems);
(c) culture for one week added with growth factors twice a week;
(d) evaluation of sterility, vitality, cell senescence, chromosomic arrangement, immunophenotype characteristics in haematopoietic and monoendothelial senses, during ex-vivo culture, according to present European regulations on cell therapy.
(e) preparation of the cell therapy product depriving the cells, prior to infusion, of serum for 3 hours and then carrying out successive washing in physiological solution;
(f) resuspension of the cell therapy product in 10 ml of physiological solution;
(g) intracoronary injection with instruments for cardiac catherisation and heart surgery.
(h) complete elution of the cell therapy product in the injection site washing with a sterile syringe the system of tubes which allow the fluidity of the instruments for angioplasty, with 1 ml of sterile physiological solution sterile to guarantee a thorough washing of the system with a recuperation of all the cells therein contained.

The biological tissue used to isolate the mesenchymal stem cells is a quantity of 150 ml of autologous bone marrow from patients in the second day after acute infarction or via the standard procedure in epidural anaesthesia with Bupivacarine 0.5% at level L3-L4 via repeated punctures of the posterior iliac crest (Bacigalupo A, 1992).

### EXAMPLES

### Example 1 - Isolation and expansion of CD34+ haematopoietic bone marrow stem cells

The isolation and expansion protocol of haematopoietic stem cells follows the requirements as laid down in the *"*Guide lines for the beginning of phase I/II clinical studies with living human cells for somatic cell therapy" (Italian Health Ministry Report, volume 12, 1997) and in *"*Guide lines for tissue engineering and cell therapy (Italian Health Ministry Report, volume 12, 1999) and "ISTSAN Report" (01/22, 2001).
The CD34⁺ cells from the bone marrow are separated by immunomagnetic selection using the commercial CliniMacs system (Miltenyi Biotec).
The recuperated CD34⁺ cells are placed in flasks (Corning-Costar, Celbio), in liquid culture in IMDM medium (Iscove Modified Dulbecco's Medium, Invitrogen), without adding antibiotics, added with 10% of foetal bovine serum treated for 30 minutes at 56°C (FBS Defined-Hyclone, EMEA approved) at a concentration of 5-10x10⁴/mL, incubated at 37°C and with 5% of CO₂ with the following carrier-free ex-vivo cytokines: IL-6 (10 ng/mL, R&D systems), FL (50 ng/mL R&D systems), TPO (10 ng/mL R&D systems), SCF (50 ng/mL R&D systems). The expansion culture system lasts 1 week. Cytokines are added every 3/4 days.
The expanded cells are carefully characterised and analysed with the following tests, according to the European cell therapy regulations in force:
• Evaluation of sterility (no contamination with anaerobe, aerobe bacteria, or mycetes) with routine bacteriological analysis using VITAL (Biomerieux, F) on 500µl of cell supernatant (Huyghe G, Demayer S, Mouillot L et al. Validation of the VITAL blood culture system, for the sterility control of labile blood products. P1072 ECCMIID 1997, Lausanne, CH).
• Evaluation of the absence of mycoplasma contamination by PCR analysis (Mycoplasma reagent set, Euroclone, UK): the analysis is carried out on 200 µl of cell supernatant.
• Evaluation of the absence of endotoxin bacteria contamination with a chromogenic test (Limulus Amebocyte Lysate Kinetic-K-QLC, Cambrex). This method creates a standard curve and the analysis of the samples is carried out on 100 µl of cell supernatant which are preincubated for 10 minutes at 37°C and then incubated at 37°C in Limulus Amebocyte Lysate for a maximum of 1 hour 40 minutes. The reading is carried out at 405 nm.
• Evaluation of cell vitality with Trypan Blue analysis (the cells are observed under an optical microscope using a Burker chamber) and by immunocytofluorimetric analysis for propidium iodide exclusion. For this analysis 20.000 cells were incubated for 10 minutes in 200 µl of solution containing 0.5 µg/ml of propidium iodide in PBS once and the evaluation was made on propidium iodide negative cells, 10.000 events were acquired.
• Immunological determination of markers characterising the haematopoietic stem cells (CD45⁺, CD34⁺ and CD14⁺/34⁻, CD14⁺/105⁺, CD14⁺/31, CD14⁺/KDR⁺, CD34⁺/133⁺ cells) by flow cytometry analysis. This analysis is carried out on 20.000 cells which are incubated for 20 minutes at 4°C in the dark with FITC or PE stained antibodies. After washing in PBS once the cells are resuspended in 200 µl of PBS once and analysed on an Epics-XL cytofluorimeter (Beckman Coulter), 10.000 events were acquired. The CD34 cell cytoflowrimetric count is made by using the ISHAGE protocol.
• Evaluation of the *in vitro* clonogenic potential of the committed haematopoietic progenitors (CFU-GEMM, CFU-GM and BFU-E, deemed responsible for the short term graft of the transplant) and the haematopoietic multipotent progenitors (LTC-IC progenitors upon which long term graft depends), allowing the evaluation of haematopoietic function of the expanded cell product.
• Cytogenetic test to exclude alterations acquired by the chromosomic complement (caryotype test). To analyse the caryotype at least 25 metaphses were analysed starting from 200.000 base cells and at the end of ex-vivo expansion. The cells are left in culture for 24 hours with a proliferative exogenous stimulus such as IL3 10 ng/ml and GM-CSF 50 ng/ml to the cells can divide. Then, in order to block the cells in metaphase, colcemid (100 ng/ml) is added. The cells are then washed in medium and centrifuged at 200g for 7 minutes. A hypotonic solution (KCl 0.075M), which had been preheated at 37°C, is gradually added up to a volume of 10 ml. The samples are incubated in a water bath at 37°C for 10 minutes, and centrifuged at 200g for 7 minutes. After removing the supernatant, a fixative is added drop by drop (methanol- acetic acid 3:1 purity solution) to obtain a volume of around 8 ml, keeping the test tubes shaken. The cells are centrifuged at 200g for 7 minutes and the fixative passage is repeated twice. *Preparation of slides:* after degreasing the slides, 2 drops of samples which had been resuspended in the fixative and collected with a Pasteur pipette, are dropped onto the slide. The slide is observed under a microscope to evaluate the quantity of nuclei and metaphases; should there be high density, the sample is diluted with fixative. Four slides are prepared for each sample. The slides are then incubated in a thermostat at 55°C overnight. *G Banding tecnique:* the slides are immersed for 20 minutes in SSC buffer solution twice in a water bath at 55°C, then washed under running water and left to dry. *Slide colouring:* the slides are immersed for 10 minutes in 100 ml of 5% Giemsa solution, then quickly washed by dipping them for a few seconds in 100 ml of Buffer phosphate (50 ml of Na₂HPO₄ + 50 ml of KH₂PO₄) and 100 ml of distilled water. The slides are then left to dry. *Observation of the slides:* the metaphase analysis is carried out under an optical microscope using MacKtype software (Nikon).
• Measure of cell senescence by Flow-FISH flow cytometry analysis of telemere length on at least 400.000 base cells and at the end of *ex-vivo* expansion. The cells are washed with a PBS solution / 0.1% BSA, placed in 1.5 ml Eppendorf tubes (6x10⁵ cells per sample), briefly centrifuged at maximum speed, and resuspended in PBS / 0.1% BSA. The sample is then divided equally into two Eppendorf tubes and centrifuged for 15 seconds at 13000 rpm. The cell pellet is then resuspended in a hybridisation solution containing 70% of deionised formamide, 20 mM of Tris pH 7.0, 1% BSA and 0.3 µg/ml of a PNA probe (peptid nucleic acid, CCCTAA), specific for the FITC coniugated telomeres. The samples are set at 82°C for 10 minutes to denature the DNA and hybridised for 2 hours at room temperature in the dark. Finally, the sample are centrifuged for 7 minutes at 16°C at 3000 rpm and washed twice with 1 ml of solution containing 70% of deionised formamide, 20 mM of Tris pH 7.0, 0.1% BSA and 0.1% of Tween 20 (centrifuged for 7 minutes at 16°C at 3000 rpm) and once with PBS/0.1% BSA/0.1% of Tween 20 (centrifuged for 5 minutes at 16°C at 3000 rpm). The cells are then resuspended (10⁵ cells/100 µl) in PBS 0.1% BSA containing RNasi (10 µg/ml) and propidium iodide (0.06 µg/ml), incubated for 2-4 hrs at room temperature and then analysed by flow cytometry.

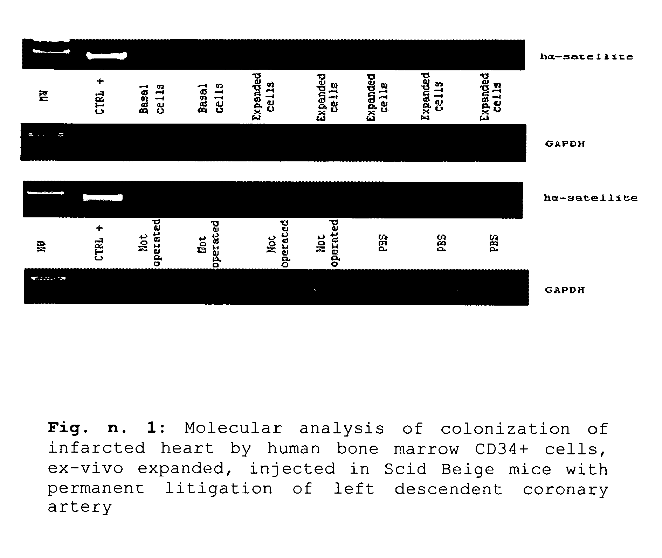

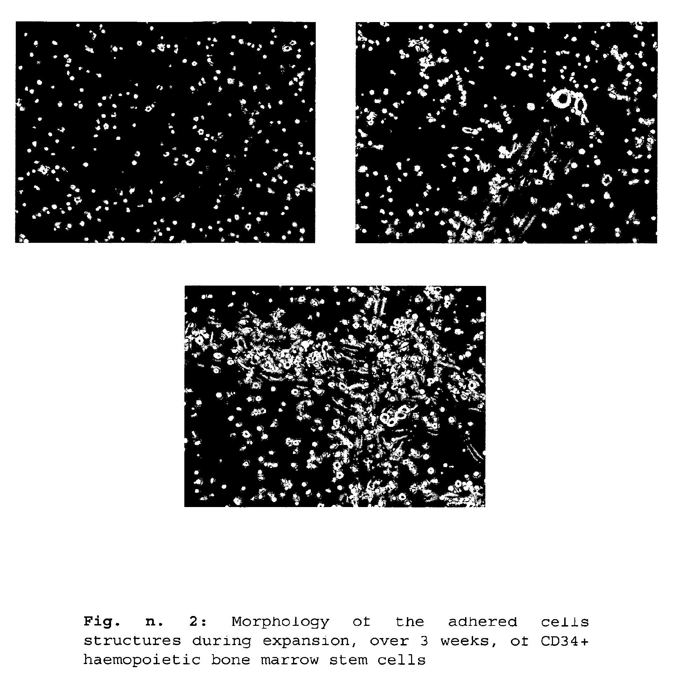

The results of the expansion process of the method concerning our invention are summarised in Tables 1, 2, 3, 4 and Figure 1 and 2:
The cell proliferation data, the increase of the absolute number of CD34⁺, CFC, and LTC-IC cells show how the one week culture procedure does not lead to a significant increase in bone marrow stem cells but it does seem, however, to modify the immunophenotype arrangement (Tables 1A and 1B). The immunophenotype analysis of the marrow cells at time zero and after a week of culture would thus seem to show that the expanded population contains a greater number of monocyte progenitors addressed towards the endothelial line (CD34⁺CD133⁺; CD14⁺CD34⁻; CD14⁺CD105⁺; CD31⁺CD105⁺; CD31⁺KDR⁺cells) (Table 3). Molecular analyses conferman these flow cytometry data (Table 4). Intramiocardial injection experiments of CD34⁺ stem cells isolated from human bone marrow and ex-vivo expanded, in Scid Beige mouse model with permanent ligation of left descendent coronary artery, demonstrated that after 4 weeks from injection, human cells are able to colonies the damaged organ. The analysis was performed by PCR for the α-satellite of human chromosome 17 (Figura 1). Furthermore, by increasing the culture by over a week, elongated structures made up of spindle-shaped cells from haematopoietic cells cultured in suspension medium, form and adhere to the culture flasks (Figure 2). The immunophenotype analysis (Table 5) of these structures shows their monoendothelial nature, suggesting that this culture system permits the maintenance and expansion of the marrow precursor common to the haemoendothelial line.
The caryotype analysis highlights how the culture process does not modify the chromosomic arrangement. The telomere length analysis shows the culture process has no effect on early cell aging (Table 2).
The sterility tests carried out each week show how the culture process was always carried out under sterile conditions. The research analysis for mycoplasma contamination was negative throughout the expansion process. The LAL test shows how the endotoxin level was within normal values (<0,5 EU/ml) throughout (Table 2). Our previous studies with the ELISA method for other protocols show how extensive washing with PBS, before administering the product, limit the quantity of bovine protein to values of less than 2 ng
Moreover, all laboratory procedures were evaluated by numeric indicators which allow the whole quality control system to be monitored, according to the requirements of ISO 9001:2000 regulations and JACIE Standards.

**Table 1A. Summary of data obtained during the expansion process.**

| | **INITIAL VOLUME** | **INITIAL %CD34⁺** | **INITIAL CD34⁺** | **%CD34⁺ AFTER CLINIMACS** | **CD34⁺ AFTER CLINIMACS** | **%CD34⁺ AFTER EXPANSION** | **CD34⁺ AFTER EXPANSION** | **MNC AFTER EXPANSION** |
|---|---|---|---|---|---|---|---|---|
| **#VALIDATION 1** | 50 ml | 0.48% | 2.5x10⁵ | 64% | 0.61x10⁶ | 38.8% | 2.7x10⁶ | 7x10⁶ |
| **#VALIDATION 2** | 44 ml | 0.81% | 3.56x10⁶ | 87% | 1.6x10⁶ | 34% | 3.6x10⁶ | 16x10⁶ |
| **#VALIDATION 3** | 50 ml | 1.46% | 12x10⁶ | 72% | 7.8x10⁶ | 34% | 3.5x10⁶ | 9.6x10⁶ |
| **#VALIDATION 4** | 63 ml | 0.69% | 4.76x10⁶ | 77% | 2.5x106 | 31.4% | 2.5x10⁶ | 7.8x10⁶ |
| **#VALIDATION 5** | 65 ml | 0.82% | 9.3x10⁶ | 86% | 6x10⁶ | 38% | 5.01x10⁶ | 13.2x1 0⁶ |

**Table 1B. Comparison between the number of cells obtained during the expansion process from 50 ml of bone marrow and the estimated number from 150 ml of bone marrow.**

| | **BASAL CD34⁺ obtained from 50 ml of bone marrow** | **BASAL CD 34⁺ estimated from 150 ml of bone marrow** | **CD34⁺ AFTER EXPANSION obtained from 50 ml of bone marrow** | **CD34⁺ AFTER EXPANSION estimated from 150 ml of bone marrow** |
|---|---|---|---|---|
| **#VALIDATION 1** | 2.5x10⁶ | 7.5x10⁶ | 2.7x10⁶ | 8.1x10⁶ |
| **#VALIDATION 2** | 3.56x10⁶ | 10.7x10⁶ | 3.8x10⁶ | 12.9x10⁶ |
| **#VALIDATION 3** | 12x10⁶ | 36x10⁶ | 3.5x10⁶ | 10.5x10⁶ |
| **#VALIDATION 4** | 4.76x10⁶ | 14.3x10⁶ | 2.5x10⁶ | 5.9x10⁶ |
| **#VALIDATION 5** | 9.3x10⁶ | 27.9x10⁶ | 5.01x10⁶ | 11.7x10⁶ |
| **MEDIA** | | 19.3 x10⁶ | | 9.8x10⁶ |

**Table 2. Summary of data obtained during the expansion process.**

| | **STERILITY** | **MYCOPLASOLA** | **ENDOTOXIN LEVEL** | **VITALITY** | **TOTAL INCREASE OF CD14⁺ NUMBERS** | **TOTAL OF CD34⁺ NUMBARES** | **TOTAL INCRASE OF CPCNUMBERS** | **TOTAL INCREASE OF LTC-IC NUMBERS** | **KARYOTYPE** | **TELOMERES** |
|---|---|---|---|---|---|---|---|---|---|---|
| ***VALIDATION1** | | | | | | | | | | |
| BASAL | NC | NC | ND | 93.0% | 1X | 1X | 1X | 1X | NORMAL | 7.4 |
| +1 WEEK | NC | NC | <0.4EU/ml | 99% | 7.3X | 4.4X | 2.1X | 0.2X | NORMAL | 9.3 |
| **#VALIDATION2** | | | | | | | | | | |
| BASAL | NC | NC | ND | 95.4% | 1X | 1X | 1X | 1X | NORMAL | 6 |
| +1 WEEK | NC | NC | <0.4EU/ml | 99% | 3.1X | 0.6X | 2.1X | 1X | NORMAL | 7.5 |
| **#VALIDATION3** | | | | | | | | | | |
| BASAL | NC | NC | ND | 96.24 | 1X | 1X | 1X | 1X | NORMAL | 7 |
| +2 WEEK | NC | NC | <0.4EU/ml | 99% | 1.1X | 0.6X | 1.2X | 0.2X | NORMAL | 7.5 |
| **#VALIVATION4** | | | | | | | | | | |
| BASAL | NC | NC | ND | 96.4% | 1X | 1X | 1X | 1X | NORMAL | 5.9 |
| +1 WEEK | NC | NC | <0.4EU/ml | 99% | 2.4X | 1X | 0.8X | 2.5X | NORMAL | 6.5 |
| **#VALIDATION5** | | | | | | | | | | |
| BASAL | NC | NC | ND | 97% | 1X | 1X | 1X | 1X | NORMAL | 7.4 |
| +1 WEEK | NC | NC | <0.4EU/ml | 99.5% | 1.9X | 0.5X | 0.5X | 1.4X | NORMAL | 6.7 |

| | | | | | | | | | | |
|---|---|---|---|---|---|---|---|---|---|---|
| NC = no contamination; ND = not determined | | | | | | | | | | |

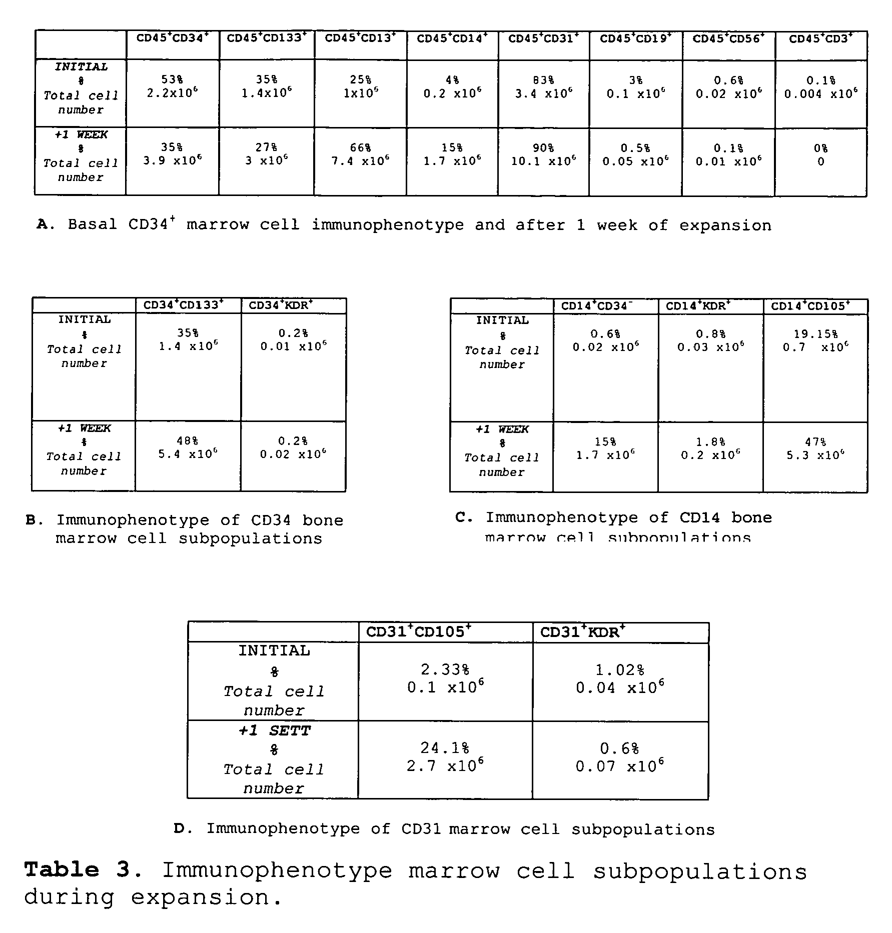

**Table 4. Real-Time PCR molecular analysis of the cell subpopulations after 1 week of expansion**

| | **INITIAL** | **+1 WEEK AFTER EXPANSION** |
|---|---|---|
| | | **-ΔΔCt** |
| CD34 | ○ | 0.193626 |
| CD133 | ○ | 0.562431 |
| CD14 | ○ | 4.890455 |
| CD31 | ○ | 0.871634 |
| CD105 | ○ | 2.242903 |
| KDR | ○ | -3.33126 |

**Table 5: Immunophenotype characterisation of adhered cell structures formed during expansion over 3 weeks, of CD34+ haematopoietic stem cells isolated from bone marrow.**

| | **ADHERED CELL STRUCTURES** |
|---|---|
| CD45+ | 96% |
| CD14+ | 71.4% |
| CD31+ | 72% |
| KDR+ | 0.7% |
| CD29+ | 99% |
| CD105+ | 62.3% |
| CD90+ | 4.2% |
| CD73+ | 5.2% |
| CD34+ | 0.1% |

### Example 2 - The preparation of haematopoietic stem cells for transplant

After culture, three hours before infusion the culture medium with serum is removed and the cells are carefully recuperated, washed in serum free culture medium and reincubated for an hour so the cells eliminate the proteins in the serum and the cytokines. The cells are then resuspended in physiological solution and are again centrifuged at 400g for 10 minutes. The cell pellet is then resuspended and washed once more with physiological solution. After the final wash, the cells are resuspended in 10 ml of physiological solution and are ready for transplant. The cells will be delivered in a 10 ml syringe, labelled according to the Laboratory's internal procedures (PG5-labelling) and existing regulations. The cell therapy product, as a cell suspension resuspended in 10 ml of physiological solution, will be injected in the intracoronary by medical, and paramedical personnel who are specialised in haemodynamics and cardiac surgery in sterile operating rooms using instruments for cardiac catheterisms and heart surgery. An over-the-wire balloon catheter which is 0.5mm larger than the stent implanted during primary angioplasty carried out in the acute phase, will be positioned at the level of the stent and inflated at low pressure in order to block the intracoronary blood flow for 3 minutes, at the same time 3.3ml cell suspension will be distally infused at the balloon by its central lumen. This flow block infusion should facilitate the adhesion and transmigration of the infused stem cells through the endothelial barrier. After 3 minutes the balloon is deflated and blood flow is allowed for a further 3 minutes (and in general for the full reestablishment of the transitorily avascularized myocardium via an ecg and verification of the patient's subjective symptoms), before starting the successive intracoronary infusion cycle. The manoeuvre will be repeated 3 times in order to obtain complete infusion of 10 ml cell suspension. The complete elution of the cell therapy product in the injection site will be carried out by washing, with a sterile syringe, the tube system to permit the fluidity of the angioplasty instruments, with 1 ml of sterile physiological solution to guarantee a complete washing of the system with a consequent recuperation of all the cells in it.

The details to achieve this and the forms of implementation may, of course, have wide variations in respect of what has be described and illustrated without going beyond the protection of this invention, as defined by the attached claims.

## Claims

1. *Ex-vivo* expansion method under clinical grade conditions, of CD34+ haematopoietic stem cells, isolated from bone marrow, in a mixture of growth factors SCF+FL+TPO+IL-6 to allow the expansion and addressed in mono-endothelial sense, to treat ischaemic pathologies such as myocardial infarction.

2. Preparation method and injection of the cell therapy product claimed in Claim 1.

3. Pharmaceutical composition including haematopoietic stem cells obtained with the method in Claim 1 and a pharmaceutically acceptable vehicle.

4. Use of haematopoietic stem cells obtained with methods in Claim 1 to prepare a medicine to treat patients affected by ischaemic pathologies such as acute myocardial infarction.

5. Use according to Claim 4, **characterised by** the medication being administered intracoronarily with haemodynamic and heart surgery techniques.

6. Use according to Claim 5, **characterised by** the fact that this medication also includes a pharmaceutically acceptable vehicle.

7. Method of treatment of patients affected by ischaemic pathologies such as acute myocardial infarction, including the administration of a therapeutically effective quantity of haematopoietic bone marrow stem cells obtained with the method in Claim 1.

8. Treatment method according to Claim 7, **characterised by** the fact that the haematopoietic bone marrow stem cells are administered intracoronarily with haemodynamic and heart surgery techniques.
